# EUROPÄISCHE PATENTSCHRIFT

(11) **EP 0 694 314 B1**
(45) Veröffentlichungstag und Bekanntmachung des Hinweises auf die Patenterteilung: **02.10.2002**
(21) Anmeldenummer: 94111786.3
(22) Anmeldetag: 28.07.1994
(51) Int. Cl.: A61M 11/06, B05B 7/00

(54) **Inhalationsvernebler mit Behältereinsatz für das Zerstäubungsgut**
Inhalation nebuliser with detachable reservoir for the product to nebulise
Nébuliseur pour inhalation avec réservoir amovible pour le produit à nubaliser

(43) Veröffentlichungstag der Anmeldung: 31.01.1996
(73) Patentinhaber: PARI GmbH Spezialisten für effektive Inhalation, 82319 Starnberg (DE)
(72) Erfinder: Brugger, Stephan, D-82301 Starnberg (DE)
(74) Vertreter: Zangs, Rainer E., Dipl.-Ing.

(56) Entgegenhaltungen:
- EP-A- 0 170 715
- EP-A- 0 540 774
- EP-A- 0 608 176
- DE-U- 8 437 274
- FR-A- 679 362
- US-A- 3 762 409

## Beschreibung

Die vorliegende Erfindung betrifft einen Behälter nach dem Oberbegriff des Patentanspruchs 1 und 2 und einen Inhalationsvernebler nach dem Oberbegriff des Anspruchs 3 und 5.

Insbesondere für medizinische Anwendungen sind zahlreiche Bauformen für Inhalationsvernebler bekannt, mit deren Hilfe eine pulverförmige oder flüssige Substanz zerstäubt und als Aerosol für Patienten bereitgestellt werden kann.

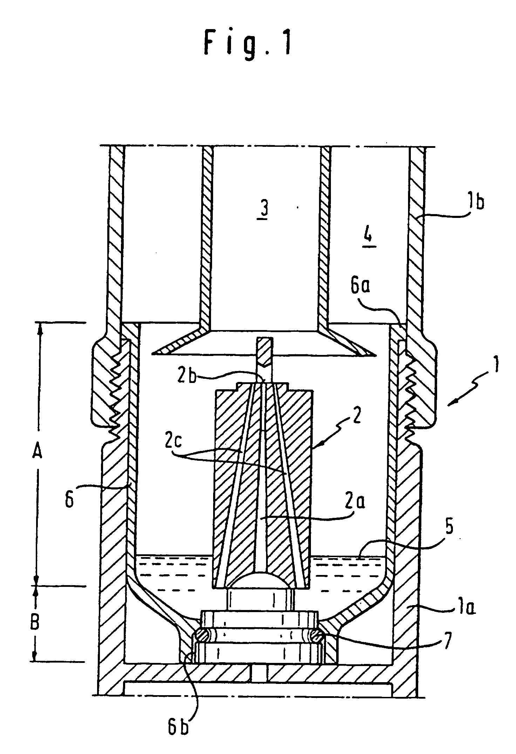

Die EP-A-0 608 176 beschreibt einen Behälter nach dem Oberbegriff des Anspruchs 1. Man beschreibt einen Mikrozerstäuber zum Vernebeln flüssiger Partikel. Der untere Teil dieser Vorrichtung besteht, wie beispielsweise aus Fig. 1 zu sehen ist, aus einem zylinderförmigen Rohr, das von einer äusseren Hülle umgeben wird. Innerhalb des zylinderförmigen Rohrs ist eine Zerstäuberdüse angeordnet. Das Rohr wird am unteren Ende durch einen separaten Verschluß abgeschlossen. Die Vorrichtung zeichnet sich insbesondere durch eine Zerkleinerungskammer aus, die eine Vielzahl von benachbarten vertieften Ringen aufweist, wie in Fig. 1 und 11 zu sehen ist.

Die US-A-3 762 409 betrifft ebenso einen Vernebler. Der Wegwerfvernebler zum Erzeugen eines Aerosols umfaßt einen abgeschlossenen Behälter, der einen Aerosolauslaß aufweist, eine Düsenanordnung, die sich in den Container erstreckt, und eine Verteileranordnung. Die Düsenanordnung umfaßt eine Gasdüse und eine Spraydüse mit beabstandeten und koaxial angeordneten Öffnungen. Die Verteileranordnung ist in dem Sprühpfad der Sprühöffnung angeordnet und umfaßt einen Nasenabschnitt, der sich in Richtung der Öffnungen erstreckt und dazu koaxial angeordnet ist. Der untere Bereich des becherförmig ausgebildeten Verneblers dient hierbei als Reservoir für das zu vernebelnde flüssige Medikament.

Ein typisches Beispiel ist in EP-A1-0 170 715 beschrieben. Der bekannte Inhalationsvernebler besitzt einen Aufnahmebereich für das Zerstäubungsgut und eine auf den Aufnahmebereich aufsetzbare Verneblerhaube, die den Zerstäubungsraum umgibt. Im Aufnahmebereich für das Zerstäubungsgut ist eine Zerstäuberdüse angeordnet, der Druckluft zugeführt wird. Die Druckluft gelangt durch einen Druckgaskanal zur Düsenmündung. Neben der Düsenmündung sind Ansaugkanäle vorgesehen, die sich im Düsenkörper bis in den Aufnahmebereich erstrecken, in dem sich das Zerstäubungsgut befindet. Dieses wird durch die beiden Ansaugkanäle angesaugt, wenn das zugeführte Druckgas aus der Düsenmündung ausströmt. Das Zerstäubungsgut wird mit dem Druckgas durchmischt und fein zerstäubt.

Bei diesem bekannten Inhalationsvernebler ist der Aufnahmebereich für das Zerstäubungsgut ein integraler Bestandteil des Gerätes selbst. Die Reinigung eines derartigen Inhalationsverneblers stellt deshalb und, weil die Abmessungen des Düsenkörpers, insbesondere der Ansaugkanäle sehr klein sind, ein Problem dar, so dass eine vollständige Reinigung nur bei sehr sorgfältiger Handhabung erzielt wird. Insbesondere bei medizinischen Anwendungen wird aber verlangt, dass das Inhalationsvernebler rückstandsfrei gereinigt werden kann, was bei bisher bekannten Inhalationsvernebler zu einem erheblichen Aufwand führt.

Eine andere Anforderung, die ebenfalls bei medizinischen Anwendungen besonderes Gewicht erlangt, besteht hinsichtlich der Dosierung des zu verabreichenden Medikaments, also des Zerstäubungsgutes. Bei den bisher bekannten Inhalationsverneblern muss die Menge des Zerstäubungsguts beim Einfüllen vom Patienten genau abgemessen werden. Eine Messung des zugeführten Medikaments während des Betriebs ist kaum möglich. Jedoch sind die Anforderungen an die Genauigkeit sehr hoch und überfordern in vielen Fällen die Möglichkeiten des Patienten.

Davon ausgehend liegt der Erfindung die Aufgabe zugrunde, einen Inhalationsvernebler zu schaffen, bei dem eine genaue Dosierung des Zerstäubungsgutes möglich ist und bei dem die Reinhaltung auf einfache Weise gewährleistet wird.

Gelöst wird diese Aufgabe durch einen Behälter gemäß Patentanspruch 1 oder 2 bzw. einen Inhalationsvernebler gemäß Patentanspruch 3 oder 5.

Im folgenden wird die Erfindung anhand von Ausführungsbeispielen genauer beschrieben. In den beiliegenden Zeichnungen zeigt:
- Fig.1: den Abschnitt eines erfindungsgemäßen Inhalationsverneblers, in dem sich die Zerstäuberdüse und der Behältereinsatz befindet, gemäß einem ersten Ausführungsbeispiel,
- Fig. 2: den erfindungsgemäßen Behältereinsatz, gemäß dem ersten Ausführungsbeispiel,
- Fig. 3: den Vorgang des Einsetzens des erfindungsgemäßen Behältereinsatzes in einen Inhalationsvernebler,
- Fig. 4: einen Inhalationsvernebler nach Entfernen des Behältereinsatzes gemeinsam mit der Zerstäuberdüse,
- Fig. 5: den erfindungsgemäßen Behältereinsatz mit einer bereits eingesetzten Zerstäuberdüse,
- Fig. 6: den Vorgang des Einsetzens eines erfindungsgemäßen Behältereinsatzes mit eingesetzter Zerstäuberdüse in einen Inhalationsvernebler,
- Fig. 7: einen erfindungsgemäßes Inhalationsvernebler im Bereich eines Zerstäuberdüsenteils gemäß einem zweiten Ausführungsbeispiel,
- Fig. 8: einen erfindungsgemäßen Behältereinsatz mit einem Zerstäuberdüsenteil gemäß dem zweiten Ausführungsbeispiel, und
- Fig. 9: einen erfindungsgemäßen Inhalationsvernebler gemäß dem zweiten Ausführungsbeispiel in zusammengesetzten Zustand.

Fig. 1 zeigt den Abschnitt eines Inhalationsverneblers 1, in dem sich die Zerstäuberdüse 2 befindet. Oberhalb der Zerstäuberdüse befindet sich in dem dargestellten Inhalationsvernebler ein Zuluftkamin 3, der zentral in dem zylindrischen Inhalationsvernebler angeordnet und selbst zylindrisch ausgebildet ist. In den Ringraum 4 um den Zuluftkamin herum wird das durch die Zerstäuberdüse erzeugte Aerosol entlassen und für das Einatmen durch den Patienten bereitgestellt. Dazu weist der Inhalationsvernebler einen in der Figur nicht gezeigten Auslaßstutzen im oberen Bereich auf. Beim Einatmen strömt Zuluft durch den Zuluftkamin in Richtung auf die Zerstäuberdüse und durch den Ringraum zum Auslaßstutzen. Dabei wird das zerstäubte Zerstäubungsgut bzw. das erzeugte Aerosol mitgenommen.

Zentral im unteren Bereich ist die Zerstäuberdüse angeordnet, die einen zentralen, sich nach oben verjüngenden Druckgaskanal 2a aufweist, der gegenüber einer Prallfläche in eine Düsenöffnung 2b mündet. Neben dem Druckgaskanal sind zwei Ansaugkanäle 2c diametral angeordnet und erstrecken sich bis in einen Bereich des Inhalationsverneblers 1, in dem sich das Zerstäubungsgut 5 befindet.

Erfindungsgemäß wird das Zerstäubungsgut von einem Behältereinsatz 6 aufgenommen, der im wesentlichen die Form eines zylindrischen Einsatzes aufweist, der an den Innenraum des Inhalationsverneblers 1 sowie die Zerstäuberdüse 2 angepaßt ist.

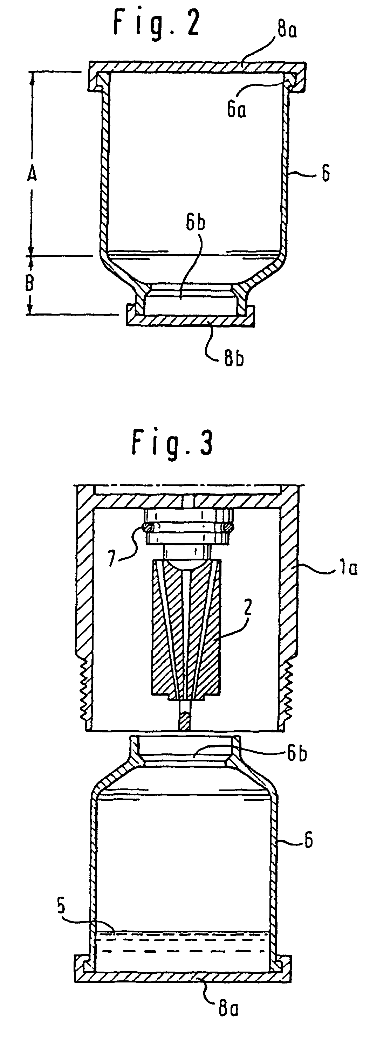

Wie in den Fig. 1 und 2 gezeigt, besteht der Behältereinsatz 6 aus einem oberen zylindrischen Abschnitt A, der dicht an der inneren Wand des Inhalationsverneblers 1 anliegt, und einem unteren Abschnitt B, mit dem eine Anpassung an die Gestalt der Zerstäuberdüse 2 erfolgt. Der obere Rand 6a des Behältereinsatzes 6 ist vorteilhaft so ausgestaltet, daß er auf dem oberen Rand eines unteren Gehäuseteils 1a des Inhalationsverneblers 1 aufliegt, wie in Fig. 1 dargestellt ist. Der Inhalationsvernebler besteht im gezeigten Beispiel aus einem unteren und einem oberen Gehäuseteil 1a bzw. 1b, die etwa mittels Schraubverbindung miteinander lösbar verbunden sind. Der sich nach unten anschließende Abschnitt B des Behältereinsatzes 6 ist im wesentlichen kegelig und erstreckt sich bis zu einer Durchtrittsöffnung 6b für die Zerstäuberdüse 2. Die Durchtrittsöffnung 6b ist so gestaltet, daß im Zusammenspiel mit einem am Fuß der Zerstäuberdüse in einer Nut vorgesehenen Dichtungsring 7 eine sichere Abdichtung zwischen Behältereinsatz 6 und Zerstäuberdüse 2 erreicht wird.

Im Inneren des Inhalatorgehäuses umschließt der Behältereinsatz 6 den Aufnahmeraum für das Zerstäubungsgut vollständig und verhindert so eine Kontaminierung des unteren Gehäuseteils 1a des Inhalationsverneblers 1 durch das Zerstäubungsgut. Denn im Bereich des Fußes der Zerstäuberdüse 2 wird durch die oben erwähnte oder andere geeignete Dichtungsmaßnahmen ein Austreten des Zerstäubungsgutes verhindert. Der obere Rand 6a des Behältereinsatzes 6 liegt auf dem unteren Gehäuseteil auf, wobei sich die umlaufende Wulst vorteilhaft bis zur Innenwand des oberen Gehäuseteils 1b erstreckt.

Der Behältereinsatz 6 kann unabhängig vom Inhalationsvernebler angefertigt und befüllt werden. In Fig. 2 ist der Behältereinsatz 6 mit jeweils einer oberen und unteren Verschlußkappe 8a bzw. 8b dargestellt. Im Inneren des so verschlossenen Behältereinsatzes 6 kann eine genau dosierte Menge eines beliebigen Zerstäubungsgutes, etwa eines Medikaments bevorratet werden. Für den Benutzer des erfindungsgemäßen Inhalationsverneblers entfällt dadurch die Aufgabe der Dosierung des Zerstäubungsgutes, da er erfindungsgemäß auf vorgefertigte, befüllte und verschlossene Behältereinsätze zurückgreifen kann und nur noch in den Inhalationsvernebler einsetzen muß.

Dazu wird vor dem Gebrauch des Inhalationsverneblers der Behältereinsatz 6 kopfüber gehalten und die Verschlußkappe 8b an der Durchtrittsöffnung 6b für die Zerstäuberdüse 2 entfernt. Das Zerstäubungsgut 5 sammelt sich dabei im Bereich der gegenüberliegenden Verschlußkappe 8a. Von oben wird dann, wie in Fig. 3 gezeigt, der untere Gehäuseteil 1a des Inhalationsverneblers 1 mit der darin angeordneten Zerstäuberdüse 2 auf den einseitig geöffneten Behältereinsatz 6 aufgesteckt, bis der Fuß der Zerstäuberdüse in der Durchtrittsöffnung 6b des Behältereinsatzes 6 zu liegen kommt und ein dichter Sitz des Behältereinsatzes 6 im Bereich des Zerstäuberdüsenfußes erreicht ist. Durch eine entsprechende Dimensionierung ist dieses Ziel ohne weiteres zu erreichen, so daß eine weitergehende Schilderung sich erübrigt.

Danach wird der untere Gehäuseteil 1a des Inhalationsverneblers mit dem eingesetzten Behältereinsatz 6 aufrecht gestellt, so daß sich das Zerstäubungsgut in dem dafür vorgesehenen Bereich des Behältereinsatzes 6 am Fuße der Zerstäuberdüse 2 sammelt, wie schon in Fig. 1 gezeigt ist. Nun kann die obere Verschlußkappe entfernt, und durch Aufsetzen des oberen Gehäuseteils der Inhalationsvernebler 1 verschlossen werden. Der Inhalationsvernebler 1 ist betriebsbereit.

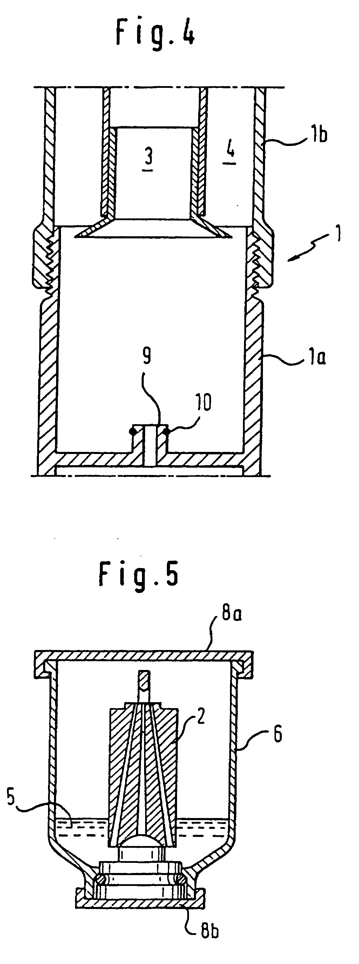

Nach dem Verbrauch des Medikaments kann der Behältereinsatz 6 wieder aus dem Inhalationsvernebler entfernt werden. Dabei ist vorteilhaft, wenn die Zerstäuberdüse 2 mit dem Inhalationsvernebler 1 nicht fest verbunden, sondern auf einen Stutzen 9 mit einem Dichtring 10 aufgesteckt, die in Fig. 4 dargestellt sind. Der Patient kann durch Ziehen an der Zerstäuberdüse 2 den Behältereinsatz 6 zusammen mit der Zerstäuberdüse 2 aus dem Inhalationsvernebler entfernen. Durch eine geeignete Dimensionierung im Zerstäuberdüsenfuß vorgesehenen Öffnung für den Stutzen 9 mit Dichtring 10 wird ein einfaches Entfernen ermöglicht, ohne daß auf die erforderliche Abdichtung verzichtet werden muß. Da der Behältereinsatz 6 zusammen mit der Zerstäuberdüse 2 aus dem Inhalationsvernebler 1 entfernt werden kann, werden diejenigen Teile des Inhalationsverneblers vollständig daraus entfernt, die besonders schlecht zu reinigen sind. Wie in Fig. 4 erkennbar, muß der Patient nur noch die weniger stark kontaminierten Teile im oberen Gehäuseteil 1b des Inhalationsvernebleres reinigen, die darüber hinaus aufgrund ihrer Form ohne größere Schwierigkeiten gereinigt werden können. Die Zerstäuberdüse 2 sowie der mit dem Zerstäubungsgut unmittelbar und über längere Zeit in Kontakt stehende Behältereinsatz 6 sind demgegenüber vom Patienten nicht zu reinigen, sondern kann zur Reinigung und Wiederbefüllung z.B. an den Hersteller des Medikaments zurückgegeben werden.

Wie aus Fig. 4 erkennbar ist, muß bevor ein neuer Behältereinsatz 6 in den Inhalationsvernebler eingesetzt werden kann, eine neue Zerstäuberdüse auf den Stutzen 9 mit Dichtring 10 aufgesteckt werden. Der Patient erhält dazu vorteilhaft sowohl einen befüllten und verschlossenen Behältereinsatz, als auch eine neue bzw. gereinigte Zerstäuberdüse in Form eines Sets. Bei der Zusammenstellung dieser beiden Bestandteile des Sets kann bei der Auswahl der Zerstäuberdüse hinsichtlich der Größe der Düsenkanäle bzw. der Düsenöffnung dem Zerstäubungsgut Rechnung getragen werden und eine besonders angepaßte Konfiguration ausgewählt werden. Dadurch wird erreicht, daß dem Patienten in dem Set stets eine auf das Zerstäubungsgut abgestimmte Zerstäuberdüse zur Verfügung gestellt wird.

Bei besonders kritischen Medikamenten ist es erforderlich, daß eine möglichst geringe Verunreinigung stattfindet. Beim oben beschriebenen Aufstecken der Zerstäuberdüse auf den Stutzen 9 mit Dichtring 10 berührt der Patient die Zerstäuberdüse mit der Hand, was ggf. zu einer Verunreinigung der Zerstäuberdüsenoberfläche ggf. sogar im Bereich der Düsen- und Ansaugkanalöffnungen erfolgt. Um eine derartige Verunreinigung zu vermeiden kann wie in Fig. 5 gezeigt ist, die Zerstäuberdüse 2 auch unmittelbar beim Herstellen in den Behältereinsatz 6 eingesteckt, der Behältereinsatz 6 mit dem zu zerstäubenden Medikament 5 befüllt und mit den Verschlußkappen 8a bzw. 8b verschlossen werden. Zwar befindet sich bei dieser Ausführungsform die Zerstäuberdüse 2 in ständigem Kontakt mit dem Zerstäubungsgut 5, bis das Zerstäubungsgut 5 verbraucht wird, jedoch stellt dies im Hinblick auf die Möglichkeiten der vorherigen Reinigung und Sterilisierung der Zerstäuberdüse 2 beim Hersteller keine besonderen Probleme dar. Insbesondere für die oben angesprochenen kritischen Medikamente ist der gesteigerte Aufwand bei der Reinigung und Sterilisierung der Zerstäuberdüse 2 gerechtfertigt, da damit die Probleme vermindert werden können, die bei einer Verunreinigung einer Zerstäubungsdüse durch unsachgemäße Handhabung auftreten. Die Durchtrittsöffnung 6b des Behältereinsatzes 6 besitzt für diese Ausgestaltung Abmessungen, die es ermöglichen, daß der Zerstäuberdüsenfuß vollständig aufgenommen wird und die Verschlußkappe 8b aufgesetzt werden kann, wie in Fig. 5 gezeigt ist. Ferner darf die Größe der Zerstäuberdüse 2 nicht über die Gesamthöhe des Behältereinsatzes 6 hinausgehen, so daß ein Verschließen mit der Verschlußkappe 8a ebenfalls möglich ist.

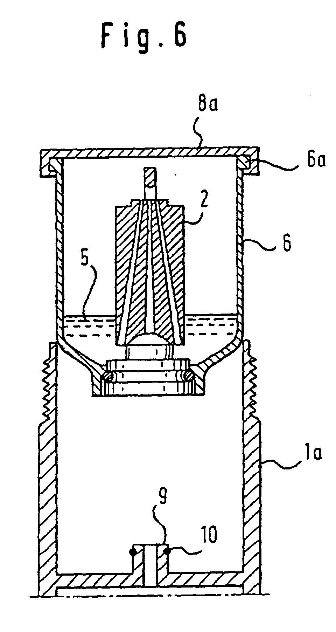

Bei diesem Ausführungsbeispiel ist ferner vorteilhaft, daß beim Einsetzen des Behältereinsatzes 6 in den unteren Gehäuseteil 1a des Inhalationsverneblers der Behältereinsatz 6 nicht auf den Kopf gestellt werden muß, bevor das Einsetzen erfolgt. Denn das Entfernen der Verschlußkappe 8b für die Durchtrittsöffnung 6b führt nicht zu einem Austreten des Zerstäubungsgutes 5, da der Zerstäuberdüsenfuß die Durchtrittsöffnung 6b dicht verschließt. Wie in Fig. 6 gezeigt, kann der mit Zerstäuberdüse ausgestattete Behältereinsatz 6 in den unteren Gehäuseteil 1a des Inhalationsvernebleres unmittelbar nach Entfernen der Verschlußkappe 8b eingesetzt werden, bis die im Zerstäuberdüsenfuß vorgesehene Öffnung für den Druckgasstutzen 9 auf den Stutzen 9 mit Dichtring 10 aufgesteckt ist. Bevor der Behältereinsatz 6 und damit die Zerstäuberdüse 2 die endgültige Position erreicht hat, wird die obere Verschlußkappe 8a abgenommen, so daß mit Erreichen der endgültigen Position der obere Rand 6a des Behältereinsatzes 6 auf dem oberen Rand des unteren Gehäuseteils 1a des Inhalationsvernebleres aufliegt. Der Inhalationsvernebler 1 wird verschlossen, indem der obere Gehäuseteil 1b aufgeschraubt wird, so daß sich der in Fig. 1 gezeigte Zustand ergibt. Der Inhalationsvernebler 1 ist betriebsbereit.

Um eine Reinigung der Zerstäuberdüse wirksam durchführen zu können, sind zahlreiche Zerstäuberdüsenformen entwickelt worden, die eine Zerlegung der Zerstäuberdüse in mehrere Bestandteile ermöglicht. Ziel dieser Bemühungen ist zumeist eine Reinigung der sehr kleinen Ansaugkanäle zu erleichtern. Die zerlegbare Ausgestaltung der Zerstäuberdüse führt aber zu einem erheblichen Aufwand bei deren Herstellung, da die Anordnung der Ansaugkanäle in Bezug auf die Düsenöffnung des Druckkanals sowie die Lage der drei Öffnungen gegenüber der Prallfläche für das Aerosol möglichst genau eingehalten werden müssen. Diese Anforderungen führen zu einer erheblichen Verteuerung der Herstellung der Zerstäuberdüse.

Bei dem im folgenden beschriebenen Ausführungsbeispiel wird der erfindungsgemäße Behältereinsatz so weitergebildet, daß er zusammen mit einem entsprechend ausgebildeten unteren Gehäuseteil eines Inhalationsverneblers auf möglichst kostengünstige Art die Vorteile der Erfindung nutzt und gleichzeitig den herstellungstechnischen Aufwand und die damit verbundenen Kosten reduziert.

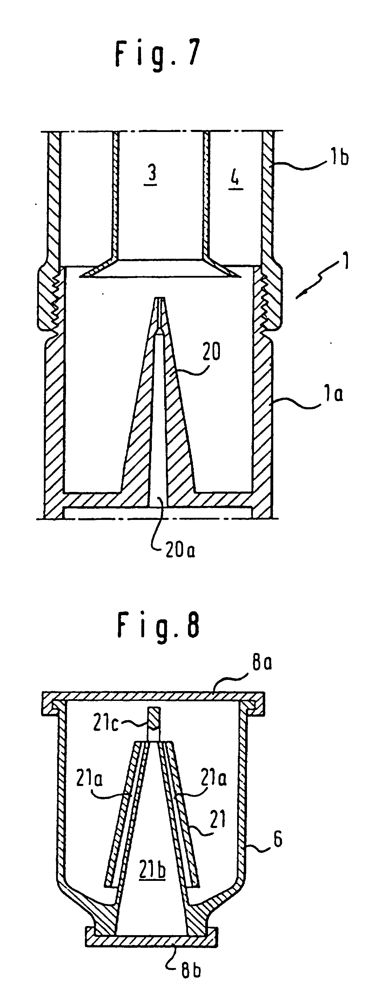

Dazu besitzt der untere Gehäuseteil 1a des Inhalationsverneblers 1 wie in Fig. 7 gezeigt ist, einen integral ausgebildeten Zerstäuberdüsenrumpf 20 in Form eines Kegels. Der Zerstäuberdüsenrumpf 20 bildet den ersten Teil der Zerstäuberdüse und dient dazu, den Druckgaskanal bis zur Düsenöffnung zu bilden. Der Druckgaskanal 20a wird beim Zerstäuben des Medikaments nicht verunreinigt, da durch das ständig strömende Druckgas ein Eindringen des Zerstäubungsgutes in den Druckgaskanal verhindert wird und eingedrungenes Zerstäubungsgut wieder aus dem Druckgaskanal hinausbefördert wird.

Vervollständigt wird die Zerstäuberdüse bei diesem Ausführungsbeispiel durch einen Zerstäuberdüsenaufsatz 21, der integral mit dem Behältereinsatz 6 ausgebildet ist, wie in Fig. 8 dargestellt ist. Der Zerstäuberdüsenaufsatz 21 bildet einen zweiten Teil der Zerstäuberdüse, in dem die beiden Ansaugkanäle 21a ausgebildet sind. Jedoch besitzt der Zerstäuberdüsenaufsatz 21 keinen Druckgaskanal, sondern einen für die Aufnahme des Zerstäuberdüsenrumpfes 20 vorbereiteten kegligen Innenraum 21b.

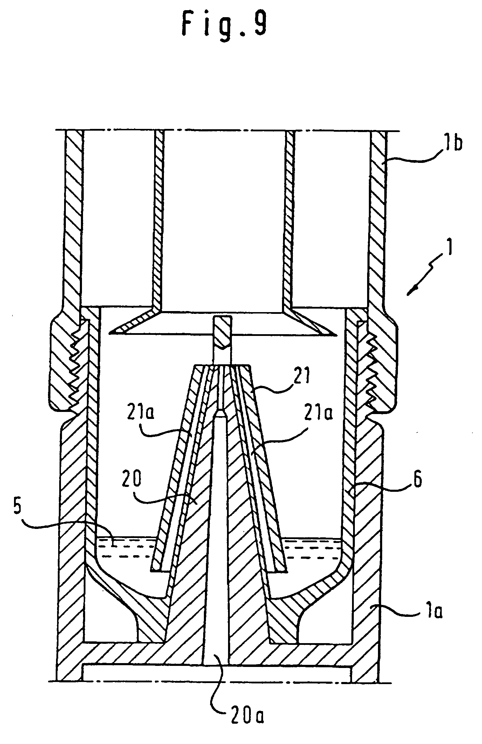

Fig. 9 zeigt den eingesteckten Zustand dieses Ausführungsbeispiels. Der kegelige Zerstäuberdüsenrumpf 20 liegt in dem kegeligen Innenraum des Zerstäuberdüsenaufsatzes 21 des Behälters 6. Die Düsenöffnung des Zerstäuberdüsenrumpfes 20 liegt in einer Ebene mit den Öffnungen der Ansaugkanäle 21a. Das Zerstäubungsgut 5 befindet sich unten im Behältereinsatz 6 und wird durch die Ansaugkanäle 21a angesaugt, wenn das in den Druckgaskanal 20a zugeführte Druckgas aus der Düsenöffnung des Druckgaskanals ausströmt.

Bei diesem Ausführungsbeispiel wird, wie in Fig. 8 gezeigt ist, der Behältereinsatz 6 durch die Verschlußkappen 8a und 8b wie in dem zuvor beschriebenen Ausführungsbeispiel verschlossen. Wenn das Luftstromsteuer 21c am Zerstäuberdüseneinsatz 21 weggelassen und die Öffnungsebene der Ansaugkanäle 21a in die unmittelbare Nähe der Verschlußkappe 8a gebracht wird, kann die untere Verschlußkappe 8b weggelassen werden. In dieser Ausgestaltung wird nämlich durch die unmittelbare Nähe der Öffnungsebene des Zerstäuberdüsenaufsatzes 21 zur Verschlußkappe 8a eine in der Regel ausreichende Abdichtung erreicht, vorzugsweise indem die Öffnungsebene die Verschlußkappe 8a berührt.

Das zweite Ausführungsbeispiel ist vorteilhaft in der Hinsicht, daß der Behältereinsatz einstückig mit dem für die Zerstäubung wesentlichen Teil der Zerstäuberdüse hergestellt wird und der stark kontaminierte Teil jeweils durch Entfernen des Behältereinsatzes aus dem Inhalationsvernebler entfernt wird. Der mit dem Zerstäubungsgut kaum oder gar nicht in Berührung kommende Zerstäuberdüsenrumpf bildet einen Bestandteil des Inhalationsverneblers und reduziert so den Herstellungsaufwand.

## Patentansprüche

1. Behälter zur Aufnahme eines Zerstäubungsguts (5), der zum Einsetzen in ein unteres Gehäuseteil (1a) eines Inhalationsverneblers nach Anspruch 3 mit einem Gehäuse (1) bestehend aus zumindest einem unteren Gehäuseteil (1a) und einem oberen Gehäuseteil (1b), das mit dem unteren Gehäuseteil (1a) lösbar verbunden ist, und einer Zerstäuberdüse (2) im unteren Gehäuseteil (1a) zum Zerstäuben eines Zerstäubungsguts (5) ausgestaltet ist, wobei der Behälter (6) eine Durchtrittsöffnung (6b) für die einzusetzende Zerstäuberdüse (2) aufweist, der Behälter **dadurch gekennzeichnet, daß** wenn der Behälter (6) in den unteren Gehäuseteil (1a) eingesetzt ist, der Zerstäuberdüsenfuß die Durchtrittsöffnung (6b) derart verschließt, dass die Durchtrittsöffnung (6b) gegen ein Austreten des Zerstäubungsguts (5) abgedichtet ist.

2. Behälter zur Aufnahme eines Zerstäubungsguts (5), der zum Einsetzen in ein unteres Gehäuseteil (1a) eines Inhalationsverneblers nach Anspruch 5 mit einem Gehäuse (1) bestehend aus zumindest einem unteren Gehäuseteil (1a) und einem oberen Gehäuseteil (1b), das mit dem unteren Gehäuseteil (1a) lösbar verbunden ist, und einer Zerstäuberdüse (2) im unteren Gehäuseteil (1a) zum Zerstäuben eines Zerstäubungsguts (5) ausgestaltet ist, der Behälter **dadurch gekennzeichnet daß** der Behälter einen Teil (21) der Zerstäuberdüse umfasst, der gemeinsam mit einem im unteren Gehäuseteil (1a) des Inhalationsverneblers vorgesehenen weiteren Teils (20) der Zerstäuberdüse diese bildet, wenn er in den Inhalationsvernebler (1, 1a) eingesetzt ist.

3. Inhalationsvernebler mit einem Gehäuse (1) bestehend aus zumindest einem unteren Gehäuseteil (1a) und einem oberen Gehäuseteil (1b), das mit dem unteren Gehäuseteil (1a) lösbar verbunden ist, und einer Zerstäuberdüse (2) im unteren, Gehäuseteil (1a) zum Zerstäuben eines Zerstäubungsguts (5) der Inhalationsvernebler **dadurch gekennzeichnet, daß** er einen im unteren Gehäuseteil (1a) einsetzbaren und herausnehmbaren Behälter (6) nach Anspruch 1, der das Zerstäubungsgut (5) enthält, aufweist, wobei der Behälter (6) eine Durchtrittsöffnung (6b) für die Zerstäuberdüse (2) aufweist und, wenn der Behälter (6) in den Inhalationsvernebler (1, 1a) eingesetzt ist, der Zerstäuberdüsenfuß die Durchtrittsöffnung (6b) derart verschließt, dass die Durchtrittsöffnung (6b) gegen ein Austreten des Zerstäubungsguts (5) abgedichtet ist.

4. Inhalationsvernebler nach Anspruch 3,
**dadurch gekennzeichnet, dass** der Behälter (6) gemeinsam mit der Zerstäuberdüse aus dem Inhalationsvernebler (1) entfernbar ist.

5. Inhalationsvernebler mit einem Gehäuse (1) bestehend aus zumindest einem unteren Gehäuseteil (1a) und einem oberen Gehäuseteil (1b), das mit dem unteren Gehäuseteil (1a) lösbar verbunden ist, und einer Zerstäuberdüse (2) im unteren Gehäuseteil (1a) zum Zerstäuben eines Zerstäubungsguts (5) der Inhalationsvernebler **dadurch gekennzeichnet, daß** er einen im unteren Gehäuseteil (1a) einsetzbaren und herausnehmbaren Behälter (6) nach Anspruch 2, der das Zerstäubungsgut (5) enthält, aufweist, wobei ein Teil (20) der Zerstäuberdüse einstückig mit dem unteren Gehäuseteil (1a) des Inhalationsverneblers (1) ausgebildet ist, und dass ein weiterer Teil (21) der Zerstäuberdüse einstückig mit dem Behälter (6) ausgebildet ist, der gemeinsam mit dem am Inhalationsvernebler (1) vorgesehenen Teil (20) der Zerstäuberdüse diese bildet, wenn der Behälter (6) in den Inhalationsvernebler (1) eingesetzt ist.

## Claims

1. Container for receiving an atomisation material (5) which is designed for insertion into a lower housing part (1a) of an inhalation nebulizer according to Claim 3 having a housing (1) consisting of at least a lower housing part (1a) and an upper housing part (1b) which is detachably connected to the lower housing part (1a) and an atomiser nozzle (2) in the lower housing part (1a) for atomising an atomisation material (5), wherein the container (6) has a passage opening (6b) for the atomiser nozzle (2) to be inserted, **characterised in that** when the container (6) is inserted into the lower housing part (1a) the foot of the atomiser nozzle seals the passage opening (6b) in such a way that the passage opening (6b) is sealed off against escape of the atomisation material (5).

2. Container for receiving an atomisation material (5) which is designed for insertion into a lower housing part (1a) of an inhalation nebulizer according to Claim 5 having a housing (1) consisting of at least a lower housing part (1a) and an upper housing part (1b) which is detachably connected to the lower housing part (1a) and an atomiser nozzle (2) in the lower housing part (1a) for atomising an atomisation material (5), **characterised in that** the container comprises a part (21) of the atomiser nozzle which jointly with a further part (20) of the atomiser nozzle provided in the lower housing part (1a) of the inhalation nebulizer forms the atomiser nozzle when it is inserted into the inhalation nebulizer (1, 1a).

3. Inhalation nebulizer having a housing (1) consisting of at least a lower housing part (1a) and an upper housing part (1b) which is detachably connected to the lower housing part (1a) and an atomiser nozzle (2) in the lower housing part (1a) for atomising an atomisation material (5), **characterised in that** the inhalation nebulizer possesses an insertable and removable container (6) according to Claim 1 which contains the atomisation material (5), wherein the container (6) has a passage opening (6b) for the atomiser nozzle (2) and when the container (6) is inserted into the inhalation nebulizer (1, 1a) the foot of the atomiser nozzle seals the passage opening (6b) in such a way that the passage opening (6b) is sealed off against escape of the atomisation material (5) .

4. Inhalation nebulizer according to Claim 3, **characterised in that** the container (6) jointly with the atomiser nozzle is removable from the inhalation nebulizer (1).

5. Inhalation nebulizer having a housing (1) consisting of at least a lower housing part (1a) and an upper housing part (1b) which is detachably connected to the lower housing part (1a) and an atomiser nozzle (2) in the lower housing part (1a) for atomising an atomisation material (5), **characterised in that** the inhalation nebulizer possesses an insertable and removable container (6) according to Claim 2 which contains the atomisation material (5), wherein a part (20) of the atomiser nozzle is constructed in one piece with the lower housing part (1a) of the inhalation nebulizer (1) and that a further part (21) of the atomiser nozzle is constructed in one piece with the container (6) which jointly with the part (20) of the atomiser nozzle provided on the inhalation nebulizer (1) forms the atomiser nozzle when the container (6) is inserted into the inhalation nebulizer (1).

## Revendications

1. Récipient destiné à recevoir une substance à pulvériser (5), qui est conçu pour être inséré dans une partie de boîtier inférieure (1a) d'un nébuliseur pour inhalation selon la revendication 3, avec un boîtier (1) se composant d'au moins une partie de boîtier inférieure (1a) et d'une partie de boîtier supérieure (1b) qui est reliée de façon amovible avec la partie de boîtier inférieure (1a), et d'une buse de pulvérisation (2) dans la partie de boîtier inférieure (1a) afin de pulvériser une substance à pulvériser (5), moyennant quoi le récipient (6) présente une ouverture traversante (6b) pour la buse de pulvérisation (2) destinée à être insérée, le récipient étant **caractérisé en ce que**, lorsque le récipient (6) est inséré dans la partie de boîtier inférieure (1a), la base de la buse de pulvérisation ferme l'ouverture traversante (6b) de telle sorte que l'ouverture traversante (6b) soit placée hermétiquement contre une sortie de la substance à pulvériser (5).

2. Récipient destiné à recevoir une substance à pulvériser (5), qui est conçu pour être inséré dans une partie de boîtier inférieure (1a) d'un nébuliseur pour inhalation selon la revendication 5, avec un boîtier (1) se composant d'au moins une partie de boîtier inférieure (1a) et d'une partie de boîtier supérieure (1b) qui est reliée de façon amovible avec la partie de boîtier inférieure (1a), et d'une buse de pulvérisation (2) dans la partie de boîtier inférieure (1a) afin de pulvériser une substance à pulvériser (5), le récipient étant **caractérisé en ce que** le récipient comprend une partie (21) de la buse de pulvérisation qui forme celle-ci conjointement avec une autre partie (20) de la buse de pulvérisation prévue dans la partie de boîtier inférieure (1a) du nébuliseur d'inhalation, lorsqu'il est inséré dans le nébuliseur pour inhalation (1, 1a).

3. Nébuliseur pour inhalation avec un boîtier (1) composé d'au moins une partie de boîtier inférieure (1a) et d'une partie de boîtier supérieure (1b), qui est reliée de façon amovible avec la partie de boîtier inférieure (1a), et d'une buse de pulvérisation (2) dans la partie de boîtier inférieure (1a) pour pulvériser une substance à pulvériser (5), le nébuliseur pour inhalation étant **caractérisé en ce qu'**il présente un récipient (6), selon la revendication 1, pouvant être inséré dans la partie de boîtier inférieure (1a) et retiré de ce dernier, lequel récipient contient la substance à pulvériser (5), moyennant quoi le récipient (6) présente une ouverture traversante (6b) pour la buse de pulvérisation (2) et, lorsque le récipient (6) dans le nébuliseur pour inhalation (1, 1a) est inséré, la base de la buse de pulvérisation ferme l'ouverture traversante (6b) de telle sorte que l'ouverture traversante (6b) soit placée hermétiquement contre une sortie de la substance à pulvériser (5).

4. Nébuliseur pour inhalation selon la revendication 3, **caractérisé en ce que** le récipient (6) peut être enlevé du nébuliseur pour inhalation (1) conjointement avec la buse de pulvérisation.

5. Nébuliseur pour inhalation avec un boîtier (1) se composant d'au moins une partie de boîtier inférieure (1a) et d'une partie de boîtier supérieure (1b), qui est reliée de façon amovible avec la partie de boîtier inférieure (1a), et d'une buse de pulvérisation (2) dans la partie de boîtier inférieure (1a) pour pulvériser une substance à pulvériser (5), le nébuliseur pour inhalation étant **caractérisé en ce qu'**il présente un récipient (6) selon la revendication 2 qui peut être inséré dans la partie de boîtier inférieure (1a) et être retiré de celle-ci, lequel récipient contient la substance à pulvériser (5), moyennant quoi une partie (20) de la buse de pulvérisation est constituée d'un seul tenant avec la partie de boîtier inférieure (1a) du nébuliseur pour inhalation (1), et **en ce qu'**une autre partie (21) de la buse de pulvérisation forme un seul tenant avec le récipient (6), qui, conjointement avec la partie (20) de la buse de pulvérisation prévue sur le nébuliseur pour inhalation (1), forme celle-ci lorsque le récipient (6) est inséré dans le nébuliseur pour inhalation (1).
